# EUROPÄISCHE PATENTANMELDUNG

(11) **EP 3 018 457 A1**
(43) Veröffentlichungstag der Anmeldung: **11.05.2016**
(21) Anmeldenummer: 15400035.0
(22) Anmeldetag: 29.07.2015
(51) Int. Cl.: G01G 19/50, A61B 5/107, G01B 3/04

(54) **VORRICHTUNG ZUR MESSUNG EINER KÖRPERLÄNGE**

(30) Priorität: 05.11.2014 DE 102014016467
(71) Anmelder: seca ag, 4153 Reinach BL 1 (CH)
(72) Erfinder: Großmann, Jan-Erik, 22527 Hamburg (DE); Hibbing, Harald, 22393 Hamburg (DE); Huß, Kirsten, 22926 Ahrensburg (DE)
(74) Vertreter: Patentanwälte Klickow & Partner Partnerschaftsgesellschaft mbB

(57) **Zusammenfassung**

Die Erfindung betrifft eine elektronische Längenmessvorrichtung zur Unterstützung einer keimarmen Situation aufweisend ein Profil in einer im Wesentlichen vertikalen Ausrichtung und einem an dem Profil längsverschiebbaren Kopfschieber als Messmittel, sowie wenigstens eine elektronische Längenwertaufnahme die mit dem längsverschiebbaren Kopfschieber derart zusammenwirkt, dass die Ermittlung eines Längenmesswertes unterstützt wird, wobei eine Elektronik zur Längenwerterfassung durch drahtlose Kommunikation mit dem längsverschiebbaren Kopfschieber zusammenwirkt und ein Steuereinheit die Anforderung von Messwerten unterstützt.

## Beschreibung

Die Erfindung betrifft eine elektronische Längenmessvorrichtung zur Unterstützung einer keimarmen Situation, aufweisend ein Profil in einer im Wesentlichen vertikalen Ausrichtung und einem an dem Profil längsverschiebbaren Kopfschieber als Messmittel, sowie wenigstens eine elektronische Längenwertaufnahme, die mit dem längsverschiebbaren Kopfschieber derart zusammenwirkt, dass die Ermittlung eines Längenmesswertes unterstützt wird.

Bekannte elektronisch arbeitende Längenmessstäbe sind infolge der teilweise hohen Anforderungen an die Präzision der Längenmesswerte durch komplexe elektromechanische Konstruktionen realisiert. Die Längenwerterfassung und Signalgenerierung beruht meist darauf, dass ein Kopfschieber entlang eines längenskalierten Profils verschiebbar angeordnet ist und dessen Position durch verschiedene bekannte physikalische Prinzipe detektierbar ist.

So werden beispielsweise kapazitive Sensoren oder Infrarotsensoren zur Längenmesswerterfassung eingesetzt, welche praktisch eine Distanzmessung längs des Verschiebeweges des Kopfschiebers darstellen. Dabei können die Sensoren im beweglichen Teil, d.h. dem Kopfschieber oder im feststehenden Teil, d.h. dem Profil des Längenmessstabs, untergebracht sein. Entscheidend für die fehler- und störungsfreie Messung ist jedoch, dass die Positionierung und Orientierung des Kopfschiebers in jedem Fall ein möglichst gleichmäßiges Spaltmaß zwischen bewegtem und fixem Teil erfordert, um ein störungsfreies Messen zu ermöglichen.

Es können auch weitere physikalische Messprinzipe, bei welchen auf die genaue Positionierung und Orientierung des Kopfschiebers relativ zum Profil oder des Profils relativ zu anderen Bauteilen des Längenmessstabes verzichtet werden kann Anwendung finden. Beispielsweise kann das Messprinzip beruhend auf dem Abspulen von Lochbändern im Inneren des Profils genutzt werden, wenn die Messergebnisqualität nicht höchsten Anforderungen genügen muss.

Soll eine hohe Messgenauigkeit erreicht werden, kann es erforderlich sein, den Längenmesswert über eine definierte Zeitspanne zu messen und zu mitteln. Weiterhin kann die Messung für die Bedienperson erleichtert werden, wenn das Längenmessgerät die Hold-Funktion bereitstellt. Bei der bekannten Messgeräten zueignen Hold-Funktion handelt es sich um die gerätespezifische Eigenschaft, einen ermittelten Messwert in der Anzeige einzufrieren, um z.B. an schwer zugänglichen Stellen genau zu messen oder einen fortlaufend gemessenen Messwert in einem idealen Moment, in dem alle Messungsrandbedingungen erfüllt sind festzuhalten. Man spricht dann davon, dass der Messwert in diesem Moment durch die Bedienperson angefordert wird. In einer anderen Definition der Hold-Funktion wird ein Messwert automatisch eingefroren, wenn über einem definierten Zeitintervall ein stabiler, d.h. weitgehend konstanter Messwert mit Schwankungen nur innerhalb eines wählbaren oder voreingestellten Toleranzbereiches gemessen wurde.

Eine Vorrichtung zur Erfassung des Umfangs eines Körperteils ist in der DE29717849U1 beschrieben, bei dem ein Sensor den Längenauszug des Maßbandes aus einem Gehäuse und anzeigt oder ggf. drahtlos überträgt.

Insbesondere bei der Verwendung von Messgeräten in Krankenhäusern, bei der Intensivmedizinbetreuung oder im Umfeld der Altenpflege, müssen keimarme oder keimfreie Situationen zumindest angestrebt werden. Es ist besonders wichtig, in diesen räumlichen Anwendungsgebieten die Möglichkeiten zu reduzieren, dass sich Krankheitserreger aufhalten können. Gleiche Anforderungen werden an besonders empfindliche Produktionsumfelder, beispielsweise die Arznei- oder Lebensmittelherstellung, gestellt.

Längenmessvorrichtungen, die in diesem Umfeld zum Einsatz kommen sollen, müssen derart konstruiert und gestaltet sein, dass Keime, Erreger und andere mikrobiologische Lebewesen keine oder geringe Möglichkeiten des Aufenthaltes vorfinden.

Von zunehmender Wichtigkeit ist es, statt der keimabtötenden Reinigung die Vermeidung von Keimen und sonstigen Organismen zu realisieren, da eine zunehmende Resistenz dieser Organismen gegenüber Medikamenten und Reinigungsmitteln zu beobachten ist.

Es ist daher Aufgabe der Erfindung eine Längenmessvorrichtung bereitzustellen, die eine keimfreie Situation unterstützt oder zumindest anstrebt, ohne dass die Ergebnisqualität der ermittelten Messwerte nennenswert zu beeinträchtigen.

Erfindungsgemäß wird diese Aufgabe gelöst dadurch, dass eine elektronische Längenmessvorrichtung derart ausgebildet ist, dass die Anzahl der Bauteile verringert und die Vorrichtungsoberfläche minimiert ist.

Insbesondere von außen zugängliche Kabel, Öffnungen und taschenförmige Oberflächenausbildungen werden vermieden. Gleiches gilt für Spalte und spielbehaftete Kontaktflächen. Die Erfindung erkennt weiterhin dass eine keimreduzierende Reinigung unterstützt werden kann, wenn Bauteile, welche gegenüber der jeweiligen geeigneten Reinigungsmethode nicht standfest sind, leicht entfernbar und ggf. einer schonenderen Entkeimung durch eine alternative Entkeimungsmethode zugeführt werden können.

Die Erfindung unterstützt den keimreduzierenden Grundaufbau einer elektronischen Längenmessvorrichtung bestehend aus den Komponenten Elektronik zur Längenwerterfassung, Profil zur Führung des beweglichen Kopfschiebers und dem Kopfschieber und ggf. einem Basiselement dadurch, dass alle verwendeten Werkstoffe und Oberflächenbeschaffenheiten zum einen der Ansiedelung von Keimen entgegenwirken und zum anderen die keimabtötende Reinigung unterstützt. Die Längenwerterfassung ist das eigentliche Messmittel. Sie sitzt vorzugsweise im Fußpunkt des Statometers und ermittelt berührungslos die Position des Kopfschiebers. Der Kopfschieber enthält keine elektronischen Komponenten zur Längenwertaufnahme. Die Position des Kopfschiebers wird von der elektronischen Längenwerterfassung, die beispielsweise im Fußpunkt des Profils sitzt, berührungslos erfasst. Die geometrische Ausbildung des elektronischen Langenmessstabes ist hinsichtlich möglichst geringer Oberflächen optimiert.

Um die möglichst kleine und abgeschlossene Oberfläche zu unterstützen, wird auf außenliegende, mechanische Messwertermittlungen und -anzeigen weitgehend verzichtet und überwiegend eine innenliegende, elektronisch arbeitende Längenmessung realisiert. Die Längenwertermittlung im Fußpunkt des Statometers wird vom Benutzer durch den Drahtlossender dahingehend gesteuert, dass sie einen über die Zeit stabilen Längenwert ermittelt (Holdwert) und diesen anschließend an eine elektronische Anzeige und/oder an ein elektronisches Datenverarbeitungssystem weiterleitet über eine elektronische Schnittstelle, die nichts mit dem Drahtlossender zu tun haben muss. Die elektronische Längenwerterfassung ist vorzugsweise innerhalb des optionalen Basiselementes und damit für Keime unzugänglich angeordnet und besitzt einen drahtlosen Empfänger, mit dem sie Steuersignale von der Steuereinheit empfangen kann. Durch die drahtlose Steuereinheit, welche vorzugsweise aus einem Sender und einer Bedieneinheit in Kombination besteht, werden Kabel, Öffnungen etc. vermieden.

In einer ersten Ausführungsvariante kann die Steuereinheit an dem Kopfschieber festgelegt sein. Dies unterstützt die Ausrichtung des zu messenden Subjektes mit gleichzeitiger Steuerung durch Auslösung an der Bedieneinheit.

In einer weiteren Ausführungsvariante kann die Steuereinheit an dem Kopfschieber lösbar festgelegt sein. Die Steuereinheit als einzig außenliegendes elektronisches Bauteil hat nicht gegenüber jeder üblichen keimtötenden Reinigungsbehandlung Standfestigkeit. Durch Entfernen der Steuereinheit von der Längenmessvorrichtung vor der Keimreinigung kann auf die verbleibende Vorrichtung jede Keimreinigungsmethode angewendet werden. Weiterhin kann eine lösbare Befestigung der Steuereinheit den praktischen Anwendungsfall unterstützen, dass zwei Bedienpersonen zusammenwirken, z.B. eine Arzthelferin richtet den zu vermessende Patienten aus, der behandelnde Arzt fordert mit der Steuereinheit, die dann quasi als Fernsteuerung genutzt wird, die Ermittlung des Holdwertes an.

Der keimreduzierende Grundaufbau der erfindungsgemäßen elektronischen Langenmessvorrichtung steht der qualitativ hochwertigen Längenmessung nicht entgegen. Realisiert wird die Längenmessung durch zwei drahtlos miteinander kommunizierende elektronische Komponenten in Form einer elektronischen Längenwerterfassung mit Empfängereinheit im Inneren der elektronischen Längenmessvorrichtung, die ggf. innerhalb des optionalen Basiselementes vorgesehen ist und einer außenliegenden, vorzugsweise lösbar am Kopfschieber angebrachten Steuereinheit, bestehend im Wesentlichen aus Sender und Bedieneinheit.

Die drahtlose Kommunikation bzw. Datenübertragung der elektronischen Komponenten kann durch bekannte Übertragungsverfahren wie Funk, W-LAN oder Blue Tooth erfolgen. Besonders bevorzugt ist jedoch die Übertragung durch Infrarotstrahlung.

Die Erfindung erkennt weiterhin, dass durch eine Kodierung der drahtlos übermittelten Informationen zwischen der Steuereinheit und der Empfängereinheit Fehler bei der Übermittlung reduziert werden können. Insbesondere fehlerhaft initiierte Anforderungen durch die Steuereinheit werden vermieden. Durch die Kodierung der Steuersignale und ein Anlernen von Sender und Empfänger kann auch vermieden werden, dass benachbart aufgestellte Statometer desselben Typs auf die Steuersignale des anderen Gerätes reagieren.

Der keimreduzierende Grundaufbau der erfindungsgemäßen elektronischen Langenmessvorrichtung strebt eine möglichst qualitativ hochwertige Längenmessung an. Aus diesem Grund werden Messungsperioden über eine definierte Zeitspanne unterstützt. Auch die Hold-Funktionen, wie oben beschrieben, werden bereitgestellt und können über die Steuereinheit angefordert werden. Wenn ein Hold-Wert ermittelt ist, kann dies bedienerfreundlich durch ein Signal optischer, akustischer oder beliebiger anderer Art angezeigt werden.

Die Erfindung nutzt weitere Maßnahmen zur Bereitstellung eines keimreduzierenden Grundaufbaus ggf. optional. Die Energieversorgung einer oder aller elektronischer Komponenten können zu diesem Zweck durch wenigstens eine Batterie realisiert werden anstelle einer Energieversorgung von extern. Dadurch werden Kabel und Öffnungen in der Oberfläche der Längenmessvorrichtung vermieden. Auch kann auf die Möglichkeit verzichtet werden, das Profil für einen möglichen Transport an wenigstens einer Stelle teilbar zu gestalten, um Keimablagerungen an der Trennstelle vorzubeugen und gleichzeitig die Stabilität zu erhöhen. Je nach gefordertem Grad des keimreduzierenden Grundaufbaus können keimabtötende Beschichtungen, geschlossenporige Lackierungen oder Werkstoffe mit geeignetem Oberflächenfinish wie z.B. gebürstetes Aluminium, blankgezogener Stahl oder glasierte Oberflächen Verwendung finden.

In den Zeichnungen sind Ausführungsbeispiele der Erfindung schematisch dargestellt. Es zeigen:
- Figur 1: eine perspektivische Vorderansicht der Längenmesseinrichtung,
- Figur 2: eine schematische, vereinfachte Darstellung der erfindungsgemäßen Längenmessvorrichtung (10).

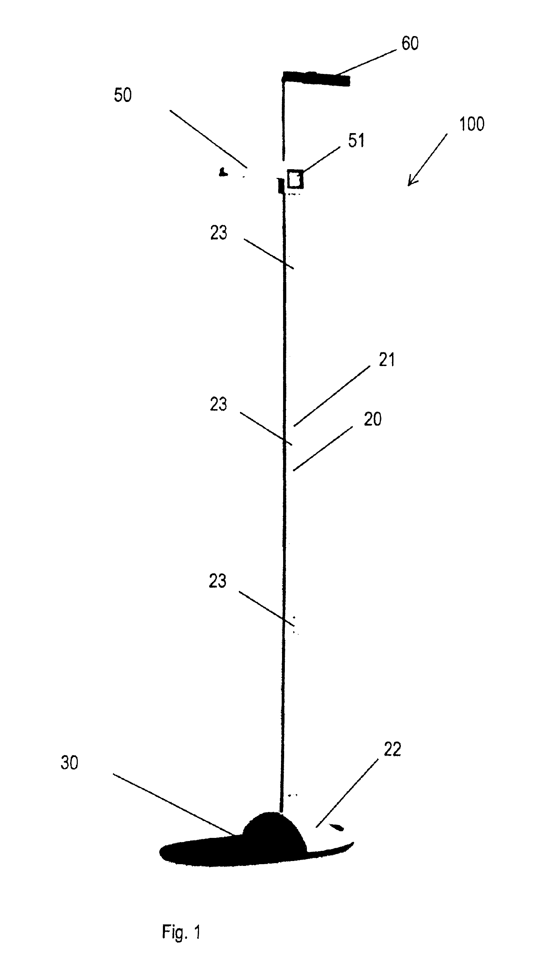

Figur 1 zeigt eine perspektivische Vorderansicht der Längenmesseinrichtung. An einem Basiselement (30) ist in einer vorzugsweise rechtwinkeligen Lage der jeweiligen Mittenachsen zueinander ein Profil (20) festgelegt, an dem ein Kopfschieber (50) vertikal beweglich angeordnet ist derart, dass mittels einer Längenmessskala (21) und der mechanischen Messwertanzeige (51) eine Längenmessinformation ermittelbar ist. Das Profil (20) ist an dem Basiselement (30) mittels eines Befestigungsschuhs (22) lösbar oder unlösbar verbunden. Das Profil (20) ist an verschiedenen Stellen, den Profilteilungen (23), in Längsrichtung zerlegbar, sodass ein kleineres Packmaß für den Transport unterstützt wird. Die erforderlichen Kontaktflächen der mechanischen Messwertanzeige (51) und der Profilteilungen (23) sind einer keimreinigenden Behandlung nur schwer zugänglich und bieten Mikroorganismen die Möglichkeit, sich dort anzusiedeln.

Um eine stabile Verbindung von Basiselement (30) und dem Profil (20) zu gewährleisten, wird häufig der Befestigungsschuh (22) als unlösbare Befestigungsausführung ausgebildet, welches üblicherweise Spalte mit von Null verschiedenen Spaltmaßen aufweisen. Wird der Befestigungsschuh (22) als lösbare Befestigungsausführung mit Taschen und Kontaktflächen ausgebildet, sind besonders gute Bedingungen gegeben, dass sich Keime und Krankheitserreger ansiedeln können. Eine optionale Wandbefestigung (60) kann vorgesehen sein, um die weniger stabile Verbindung von Basiselement (30) und dem Profil (20) durch den Befestigungsschuh (22) in der lösbaren Ausführungsvariante zu entlasten. Die Wandbefestigung ist jedoch insofern nachteilig, als dass weitere Keimflächen geschaffen werden.

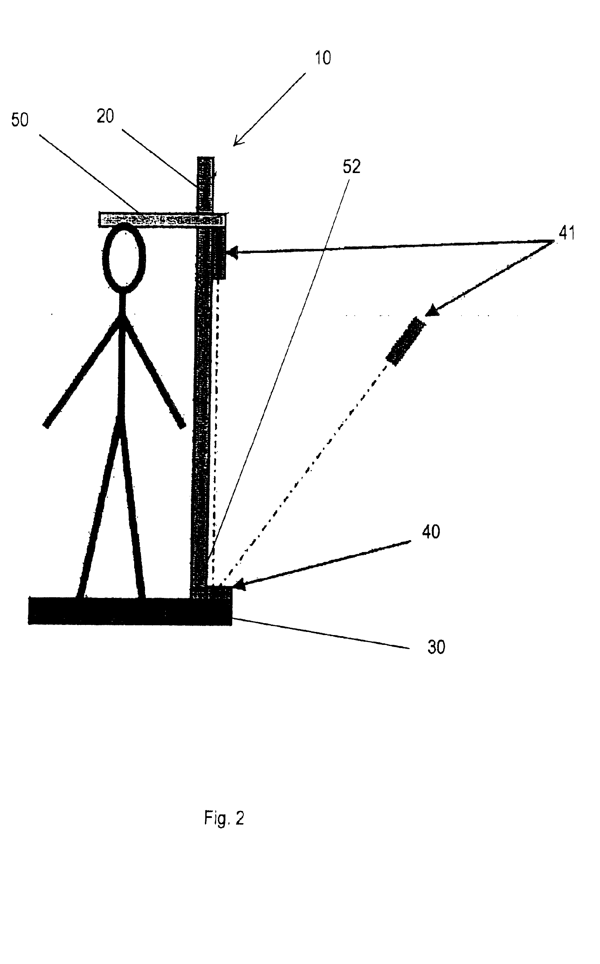

Figur 2 zeigt die schematische, vereinfachte Darstellung der erfindungsgemäßen elektronischen Längenmessvorrichtung (10). Die elektronischen Bauteile werden im Wesentlichen durch zwei drahtlos miteinander kommunizierende elektronische Komponenten in Form einer elektronischen Längenwerterfassung (52) mit Empfängereinheit (40) im Innern der elektronischen Längenmessvorrichtung (10) oder die ggf. innerhalb des optionalen Basiselementes vorgesehen ist und einer außen liegenden, vorzugsweise lösbar am Kopfschieber (50) angebrachten Steuereinheit (41), bestehend im Wesentlichen aus Sender und Bedieneinheit, gebildet. In der schematischen Prinzipskizze ist die Empfängereinheit (40) als außen liegendes Bauteil dargestellt. Vorzugsweise wird es innerhalb des Profils (20) oder des optionalen Basiselementes (30) angeordnet und auf diese Weise konstruktiv eine keimarme Gestaltung unterstützt.

Wenn die Steuereinheit (41) an dem Kopfschieber (50) angebracht ist, kann die Bedienperson die Positionierung des zu messenden Subjektes kontrollieren und korrigieren und nach dessen Ausrichtung den Holdwert anfordern, ohne die Messstelle unbeobachtet lassen zu müssen. Im Fall einer nicht an dem Kopfschieber (50) festgelegten Steuereinheit (41) kann sowohl die Bedienperson an der Längenmessvorrichtung (10) als auch eine beabstandete Person den Holdwert anfordern.

Die mechanischen Bauteile der erfindungsgemäßen elektronischen Längenmessvorrichtung (10) werden im Wesentlichen durch das Profil (20) und den an dem Profil (20) längsverschiebbaren Kopfschieber (50) sowie dem optionalen Basiselement (30) gebildet. Sowohl die Anzahl der Bauteile als auch deren Oberflächen und geometrischen Ausgestaltungen sind derart konstruiert, dass eine keimarme Situation unterstützt ist.

Im Fußpunkt des Statometers befindet sich eine elektronische Längenmesseinrichtung (52), die im Inneren des Profils (20) die Entfernung zu einem Kopfschieber (50) berührungslos messen kann (z.B. durch eine Ultraschall-Laufzeitmessung, Laserdistanzmessung, etc.). Die Längenmesseinrichtung besitzt eine elektronische Schnittstelle zu einer elektronischen Anzeige oder Datenverarbeitungseinrichtung (z.B. LCD-Display, PC, etc.), mit deren Hilfe der ermittelte Längenmesswert zur Anzeige gebracht und/oder in ein elektronisches Datenverarbeitungssystem übernommen werden kann (z.B. Patientendatenbank). Die elektronische Anzeige kann auch fester Bestandteil der Längenmesselektronik sein (z.B. integriertes Display).

Der Kopfschieber (50) dient als mechanische Einstellhilfe zur Ermittlung der Patientengröße. Er muss keine elektronischen Komponenten besitzen, es genügt wenn die Position des Kopfschiebers im Inneren des Profils erfasst werden kann. Dies kann beispielsweise durch ein an den Kopfschieber magnetisch gekoppeltes bewegliches Teil im Inneren des Profils realisiert werden, das der Position des Kopfschiebers folgt (siehe DE 10 2012 220 412 B3).

Die elektronische Längenmesseinrichtung (52) besitzt einen Drahtlosempfänger (z.B. Infrarotempfänger), der einfache Steuersignale von einer Steuereinheit mit Drahtlossender (41) empfangen kann.

Somit kann beispielsweise die Längenmessung vom Drahtlossender aus gestartet werden, oder aber auch die Ermittlung eines über einen längeren Zeitraum stabiler Messwertes angefordert werden ("Hold-Wert"), der dann von der elektronischen Längenmesseinrichtung über die elektronische Schnittstelle zur Weiterverarbeitung an eine Anzeige und/oder ein Datenverarbeitungssystem gesendet wird.

Der Drahtlossender (41) kann fest am Kopfschieber montiert sein oder auch in einer abnehmbaren und somit mobilen Fernbedienung integriert werden, sodass auch noch andere Anwendungsfälle ermöglicht werden (z.B. Arzthelfer/in richtet Patienten aus, behandelnder Arzt fordert mit der Fernbedienung den Hold-Wert der Längenmessung an).

## Patentansprüche

1. Elektronische Längenmessvorrichtung (10) zur Unterstützung einer keimarmen Situation aufweisend, ein Profil (20) in einer im Wesentlichen vertikalen Ausrichtung und einem an dem Profil (20) längsverschiebbaren Kopfschieber (50) als Messmittel, sowie wenigstens eine elektronische Längenwertaufnahme (52), die mit dem längsverschiebbaren Kopfschieber (50) derart zusammenwirkt, dass die Ermittlung eines Längenmesswertes unterstützt wird, **dadurch gekennzeichnet, dass** eine Elektronik zur Längenwerterfassung (40) durch drahtlose Kommunikation mit dem längsverschiebbaren Kopfschieber (50) zusammenwirkt und ein Steuereinheit (41) die Anforderung von Messwerten unterstützt.

2. Elektronische Längenmessvorrichtung (10) nach Anspruch 1, **dadurch gekennzeichnet, dass** die Steuereinheit (41) mit der elektronischen Längenwertaufnahme (52) und, oder der Elektronik zur Längenwerterfassung (40) drahtlos kommuniziert.

3. Elektronische Längenmessvorrichtung (10) nach Anspruch 2, **dadurch gekennzeichnet, dass** die Steuereinheit (41), die elektronische Längenwertaufnahme (52) und die Elektronik zur Längenwerterfassung (40) durch Infrarotstrecken kommunizieren.

4. Elektronische Längenmessvorrichtung (10) nach Anspruch 1, **dadurch gekennzeichnet, dass** die elektronische Längenwertaufnahme (52) im Wesentlichen durch Sensoren zur Positionserfassung des Kopfschiebers (50) und einem Sender zur drahtlosen Kommunikation gebildet ist.

5. Elektronische Längenmessvorrichtung (10) nach Anspruch 1, **dadurch gekennzeichnet, dass** die elektronische Längenwertaufnahme (52) im Wesentlichen durch eine Infrarotsender- und Empfängereinheit gebildet ist, sodass die drahtlose Kommunikation und eine Infrarotlaufzeitmessung zur Distanzmessung unterstützt ist.

6. Elektronische Längenmessvorrichtung (10) nach Anspruch 1, **dadurch gekennzeichnet, dass** die elektronische Längenwertaufnahme (52) im Innern des Kopfschiebers (50) zur Unterstützung einer keimarmen Situation angeordnet ist.

7. Elektronische Längenmessvorrichtung (10) nach Anspruch 1, und 6 **dadurch gekennzeichnet, dass** die Elektronik zur Längenwerterfassung (40) eine Infrarotsender- und Empfängereinheit aufweist, sodass die drahtlose Kommunikation und eine Infrarotlaufzeitmessung zur Distanzmessung unterstützt ist.

8. Elektronische Längenmessvorrichtung (10) nach Anspruch 1, **dadurch gekennzeichnet, dass** die Elektronik zur Längenwerterfassung (40) im Innern eines Basiselementes (30) zur Unterstützung einer keimarmen Situation angeordnet ist.

9. Elektronische Längenmessvorrichtung (10) nach Anspruch 1, **dadurch gekennzeichnet, dass** die Steuereinheit (41) im Wesentlichen aus Infrarotsender und Bedieneinheit gebildet ist.

10. Elektronische Längenmessvorrichtung (10) nach Anspruch 1, **dadurch gekennzeichnet, dass** die Steuereinheit (41) lösbar an einer Außenseite des Kopfschiebers (50) festgelegt ist.

11. Elektronische Längenmessvorrichtung (10) nach Anspruch 1, **dadurch gekennzeichnet, dass** die Energieversorgung wenigstens einer elektronischer Komponente (40, 41, 52) durch wenigstens eine innerhalb der elektronischen Längenmessvorrichtung (10) angeordnete Batterien zur Unterstützung einer keimarmen Situation realisiert ist.

12. Elektronische Längenmessvorrichtung (10) nach Anspruch 1, **dadurch gekennzeichnet, dass** die Kommunikation der elektronischen Komponenten (40, 41, 52) durch Funk, W-LAN oder Blue Tooth realisiert ist.

13. Elektronische Längenmessvorrichtung (10) nach Anspruch 1, **dadurch gekennzeichnet, dass** die zwischen wenigstens zwei elektronischen Komponenten (40, 41, 52) drahtlos übermittelten Informationen kodiert sind, sodass fehlerhafte Übermittlungen reduziert sind.

14. Elektronische Längenmessvorrichtung (10) nach Anspruch 1, **dadurch gekennzeichnet, dass** die Ermittlung und Anforderung eines Hold-Messwertes unterstützt ist.

15. Elektronische Längenmessvorrichtung (10) nach Anspruch 3 und 5, **dadurch gekennzeichnet, dass** die Ermittlung von Längenmesswerten durch die elektronische Längenwertaufnahme (52) und die Elektronik zur Längenwerterfassung (40) durch Positionserfassung des Kopfschiebers (50) mittels Sensoren und, oder Infrarotlaufzeitmessung zur Distanzmessung realisierbar ist.

16. Elektronische Längenmessvorrichtung (10) nach Anspruch 1, **gekennzeichnet durch** die Eignung, einer keimabtötenden Behandlung unterzogen werden zu können.

17. Elektronische Längenmessvorrichtung (10) nach Anspruch 16, **dadurch gekennzeichnet, dass** die keimabtötende Behandlung durch Einwirkung von H2O2, Heißdampf, hoher Temperatur, UV-Bestrahlung oder einer Kombination dessen realisierbar ist.

18. Elektronische Längenmessvorrichtung (10) nach Anspruch 1, **dadurch gekennzeichnet, dass** keimabtötende Beschichtungen und, oder geschlossenporige Oberflächen realisiert sind zur Unterstützung einer keimarmen Situation.
